(19) Europäisches Patentamt
European Patent Office
Office européen des brevets

(11) EP 1 174 110 A1

(12) DEMANDE DE BREVET EUROPEEN

(43) Date de publication:
**23.01.2002 Bulletin 2002/04**

(51) Int Cl.$^{7}$: **A61K 7/027**, A61K 7/48

(21) Numéro de dépôt: **01401905.3**

(22) Date de dépôt: **16.07.2001**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **17.07.2000 FR 0009317**

(71) Demandeur: **L'OREAL**
**75008 Paris (FR)**

(72) Inventeurs:
- **Livoreil, Aude**
  **75006 Paris (FR)**
- **Genard, Sylvie**
  **94130 Nogent Sur Marne (FR)**

(74) Mandataire: **Kromer, Christophe**
**L'OREAL - D.P.I., 6, rue Bertrand Sincholle**
**92585 Clichy Cedex (FR)**

(54) **Composition cosmétique ou pharmaceutique se présentant sous forme solide comprenant des bis-acyl-amides**

(57) La présente demande concerne une composition notamment cosmétique ou pharmaceutique se présentant sous forme solide, comprenant une phase huileuse et au moins un composé de formule (I) suivante :

R-CO-NH-A-NH-CO-R'

dans laquelle R et R' représentent un atome d'hydrogène ou une chaîne hydrocarbonée ; et A représente une chaîne hydrocarbonée.

EP 1 174 110 A1

## Description

La présente invention a trait à une composition solide notamment cosmétique ou pharmaceutique, telle qu'une composition de soin, de traitement et/ou de maquillage de la peau, y compris du cuir chevelu, et/ou des lèvres des êtres humains, ladite composition comprenant une phase grasse liquide épaissie, et se présentant notamment sous forme d'un stick ou bâton de maquillage comme un rouge à lèvres.

Dans les compositions notamment cosmétiques et dermatologiques, il est courant d'utiliser une phase grasse liquide structurée, c'est-à-dire épaissie ou gélifiée, pour obtenir la consistance souhaitée. L'épaississement des huiles (ou des phases liquides à température ambiante) permet en particulier de faciliter la prise du produit hors de son conditionnement sans perte significative, de limiter la diffusion du produit à la zone locale de traitement, de répartir le produit de façon régulière sur la zone locale de traitement ou bien encore de pouvoir utiliser le produit dans des quantités suffisantes pour obtenir l'effet cosmétique ou dermatologique recherché Ceci est notamment le cas dans les compositions solides comme les déodorants, les baumes et les rouges à lèvres, les produits anti-cerne et les fonds de teint coulés. Cet épaississement est notamment primordial pour les compositions de soin, d'hygiène ou de maquillage comme les rouges à lèvres qui doivent bien se répartir de façon homogène sur la surface locale à traiter ainsi que pour les compositions capillaires qui doivent s'étaler et se répartir de façon régulière le long des fibres kératiniques et ne pas ruisseler sur le front, la nuque, le visage ou dans les yeux.

Pour remédier à ces problèmes, on a habituellement recourt à des cires ou des charges. Malheureusement, ces cires et/ou charges ont tendance à matifier la composition et à la rendre opaque, ce qui n'est pas toujours souhaitable en particulier pour un rouge à lèvres. En effet, les femmes sont toujours à la recherche d'un rouge à lèvres sous forme d'un bâton permettant l'obtention d'un film brillant; par ailleurs, certaines compositions telles que les baumes à lèvres ou les onguents, peuvent se présenter sous forme de sticks translucides, voire transparents.

Il est également connu d'épaissir les huiles avec des épaississants polymériques. Malheureusement, les épaississants d'huiles connus doivent être utilisés en grande quantité pour obtenir un gel de viscosité élevée, par exemple supérieure à 1,3 Pa.s. Or, une trop grande quantité d'épaississant peut conférer à la composition des propriétés cosmétiques inadéquates, notamment un toucher collant et un manque de glissant, ces inconvénients pouvant être très gênants, voire rédhibitoires.

Par ailleurs, il est également connu de gélifier des compositions, notamment cosmétiques, en utilisant un gélifiant de type tri-alkyl,tri-(alkylaminocarbonyl)-cyclohexane. Ces gélifiants permettent d'améliorer la stabilité des compositions les comprenant. Toutefois, les gels obtenus sont, une fois encore, peu transparents. De plus, une grande partie de ces gélifiants ne permet pas la gélification des milieux huileux siliconés.

Enfin, il est connu d'épaissir des compositions cosmétiques à l'aide de dérivés diamidés, notamment dans les documents JP7/138555 et JP10/237034. Toutefois, les compositions cosmétiques décrites dans cet art antérieur comprennent toutes une quantité importante de cires (cire d'abeille, ozokérite ou acide héxadécanoïque notamment). La structuration et gélification du stick ne se fait donc pas uniquement grâce aux composés diamidés mais également grâce aux cires. Or l'utilisation de quantité importante de cires présente certains inconvénients, notamment en terme de matité ou d'opacité de la composition finale ainsi préparée.

De plus, il n'est pas possible d'incorporer des huiles siliconées en quantité importante dans une composition comprenant par ailleurs des cires classiques.

La présente invention a pour but de proposer l'obtention d'une composition notamment cosmétique, se présentant sous forme solide, et comprenant de préférence peu, voire pas, de cires, tout en étant susceptible, d'une part, de conserver de bonnes propriétés cosmétiques, et notamment une certaine translucidité, voire transparence, et d'autre part, de comprendre des huiles de silicone, notamment en quantité importante.

L'invention a donc pour objet une composition notamment cosmétique ou pharmaceutique se présentant sous forme solide, comprenant une phase huileuse et au moins un composé de formule (I) suivante :

R-CO-NH-A-NH-CO-R'

dans laquelle :

- R et R', identiques ou différents, représentent un atome d'hydrogène ou une chaîne hydrocarbonée saturée ou insaturée, linéaire, ramifiée ou cyclique, de préférence linéaire saturée, comprenant 1 à 22 atomes de carbone, notamment 6-18 atomes de carbone, de préférence de 10 à 14 atomes de carbone, plus préférentiellement de 11 à 13 atomes de carbone, et mieux 11 atomes de carbone, éventuellement substituée par un ou plusieurs groupements choisis parmi les groupements aryle ($-C_6H_5$), ester (-COOR" avec R" étant un groupe alkyl ayant 2 à 12 atomes de carbone), amide (-CONHR" avec R" étant un groupe alkyl ayant 2 à 12 atomes de carbone), uréthanne (-OCONHR" avec R" étant un groupe alkyl ayant 2 à 12 atomes de carbone), urée (-NHCONHR" avec R" étant

un groupe alkyl ayant 2 à 12 atomes de carbone); et/ou éventuellement comprenant 1 à 3 hétéroatomes choisi parmi O, S et N; et/ou éventuellement substituée par 1 à 4 atomes d'halogènes, notamment de fluor et/ou par 1 à 3 radicaux hydroxy, à la condition que R et R' soient différents de l'hydrogène, et

- A représente une chaîne (sous forme de radical divalent) hydrocarbonée, saturée ou insaturée, linéaire, cyclique ou ramifiée, ayant 1 à 18 atomes de carbone, notamment 2 à 12 atomes de carbone, de préférence de 4 à 10 atomes de carbone, éventuellement substitué par un ou plusieurs groupements choisis parmi les groupements aryle (-$C_6H_5$), ester (-COOR" avec R" étant un groupe alkyl ayant 2 à 12 atomes de carbone), amide (-CONHR" avec R" étant un groupe alkyl ayant 2 à 12 atomes de carbone), uréthanne (-OCONHR" avec R" étant un groupe alkyl ayant 2 à 12 atomes de carbone), urée (-NHCONHR" avec R" étant un groupe alkyl ayant 2 à 12 atomes de carbone); et/ou éventuellement comprenant 1 à 3 hétéroatomes choisi parmi O, S et N; et/ou éventuellement substituée par 1 à 4 atomes d'halogènes, notamment de fluor et/ou par 1 à 3 radicaux hydroxy.

On a en effet constaté que l'utilisation des composés de formule (I) permet de structurer et d'épaissir fortement les phases grasses liquides (ou huileuses), voire de les gélifier complètement, et ainsi d'obtenir des compositions cosmétiques stables sous forme gélifiée solide, présentant des propriétés cosmétiques satisfaisantes. Ces compositions peuvent même être exemptes de cires tout en conservant leur rigidité et leurs bonnes propriétés cosmétiques. Par ailleurs, la phase grasse liquide peut être gélifiée même si elle comprend une quantité importante d'huiles de silicone.

La composition selon l'invention présente de bonnes propriétés cosmétiques : elle n'est pas collante lors de l'application et est glissante et facile à appliquer. Elle permet l'obtention d'un film homogène et uniforme, couvrant et confortable à porter.

De plus, la composition peut avantageusement être claire, transparente ou translucide. On entend par là la définition classique donnée dans le dictionnaire. Ainsi, une composition translucide laisse passer la lumière sans permettre toutefois de distinguer nettement les contours des objets. Une composition transparente se laisse aisément traverser par la lumière et permet de distinguer nettement les objets à travers son épaisseur.

D'une manière générale, une composition transparente aura une valeur de transmittance maximum de la lumière, quelle que soit la longueur d'onde comprise entre 400 et 800 nm, à travers un échantillon de 1 cm d'épaisseur, comprise entre 35% et 100%, et de préférence d'au moins 50% (voir EP291334). Une composition translucide aura, quant à elle, une valeur de transmittance maximum de la lumière comprise entre 2 et 35%. La transmittance peut être mesurée en plaçant un échantillon de 1 cm d'épaisseur dans le rayon lumineux d'un spectrophotomètre travaillant dans les longueurs d'onde du spectre lumineux.

La composition selon l'invention comprend donc au moins un composé correspondant à la formule (I) suivante :

R-CO-NH-A-NH-CO-R'

dans laquelle :

- R et R', identiques ou différents, représentent un atome d'hydrogène ou une chaîne hydrocarbonée saturée ou insaturée, linéaire, ramifiée ou cyclique, de préférence saturée linéaire, comprenant de 1 à 22 atomes de carbone, notamment 6-18 atomes de carbone, de préférence de 10 à 14 atomes de carbone, plus préférentiellement de 11 à 13 atomes de carbone, et mieux 11 atomes de carbone, éventuellement substituée par un ou plusieurs groupements choisis parmi les groupements aryle (-$C_6H_5$), ester (-COOR" avec R" étant un groupe alkyl ayant 2 à 12 atomes de carbone), amide (-CONHR" avec R" étant un groupe alkyl ayant 2 à 12 atomes de carbone), uréthanne (-OCONHR" avec R" étant un groupe alkyl ayant 2 à 12 atomes de carbone), urée (-NHCONHR" avec R" étant un groupe alkyl ayant 2 à 12 atomes de carbone); et/ou éventuellement comprenant 1 à 3 hétéroatomes choisi parmi O, S et N; et/ou éventuellement substituée par 1 à 4 atomes d'halogènes, notamment de fluor et/ou par 1 à 3 radicaux hydroxy, à la condition que R et R' soient différents de l'hydrogène, et
- A représente une chaîne (sous forme de radical divalent) hydrocarbonée, saturée ou insaturée, linéaire, cyclique ou ramifiée, ayant 1 à 18 atomes de carbone, notamment 2 à 12 atomes de carbone, de préférence de 4 à 10 atomes de carbone, éventuellement substitué par un ou plusieurs groupements choisis parmi les groupements aryle (-$C_6H_5$), ester (-COOR" avec R" étant un groupe alkyl ayant 2 à 12 atomes de carbone), amide (-CONHR" avec R" étant un groupe alkyl ayant 2 à 12 atomes de carbone), uréthanne (-OCONHR" avec R" étant un groupe alkyl ayant 2 à 12 atomes de carbone), urée (-NHCONHR" avec R" étant un groupe alkyl ayant 2 à 12 atomes de carbone); et/ou éventuellement comprenant 1 à 3 hétéroatomes choisi parmi O, S et N; et/ou éventuellement substituée par 1 à 4 atomes d'halogènes, notamment de fluor et/ou par 1 à 3 radicaux hydroxy.

Par chaîne hydrocarbonée insaturée, on entend une chaîne qui comprend au moins une double liaison C=C, ou au moins une triple liaison C≡C, ladite chaîne pouvant bien entendu éventuellement en outre être substituée par un ou plusieurs groupements choisis parmi les groupements aryle, ester, amide, uréthanne, urée; et/ou comprendre éventuellement un ou plusieurs hétéroatomes choisi parmi O, S et N; et/ou éventuellement être substituée par un ou plusieurs atomes de fluor et/ou radicaux hydroxy.

Par chaîne hydrocarbonée comprenant un atome d'oxygène, de soufre ou d'azote, on entend notamment une chaîne hydrocarbonée comprenant un groupement carbonyle (-C=O), aminé (-NH2 ou -NH-), thiol (-SH), thioéther ou éther.

Avantageusement, A représente un radical divalent à chaîne hydrocarbonée saturée cyclique ayant de 4 à 10 atomes de carbone.

Selon un mode préféré de réalisation de la composition selon l'invention, les radicaux R et R' des composés de formule (I) sont identiques.

De préférence, les composés répondent à la formule (I) dans laquelle :

1/

- A représente un cycle (sous forme d'un radical divalent) hydrocarboné, saturé ou insaturé, de préférence saturé, mais non aromatique, éventuellement ramifié, ayant de 4 à 12 atomes de carbone, notamment de 5 à 7 atomes de carbone, éventuellement substitué par les substituants ci-dessus cités et/ou éventuellement comprenant 1 ou plusieurs hétéroatomes et/ou éventuellement substituée par 1 ou plusieurs halogènes et/ou radicaux hydroxy;
- R et R', identiques ou différents, représentent un atome d'hydrogène ou une chaîne hydrocarbonée saturée ou insaturée, linéaire, ramifiée ou cyclique, de préférence saturée linéaire, comprenant 10 à 16 atomes de carbone, notamment 10 à 14 atomes de carbone, ou encore de 12 à 14 atomes de carbone, et mieux de 11 atomes de carbone, à la condition que R et R' soient différents de l'hydrogène,

2/

- A représente une chaîne (sous forme d'un radical divalent) hydrocarbonée saturée, linéaire ou ramifiée, ayant de 2 à 18 atomes de carbone, notamment de 3 à 12 atomes de carbone, éventuellement substituée par les substituants ci-dessus cités, et/ou éventuellement comprenant 1 ou plusieurs hétéroatomes et/ou éventuellement substituée par 1 ou plusieurs halogènes et/ou radicaux hydroxy;
- R et R', identiques ou différents, représentent un atome d'hydrogène ou une chaîne hydrocarbonée saturée ou insaturée, linéaire, ramifiée ou cyclique, de préférence saturée linéaire, comprenant de 10 à 20 atomes de carbone, notamment de 11 à 18 atomes de carbone, de préférence de 11 à 13 atomes de carbone, et mieux 11 atomes de carbone, à la condition que R et R' soient différents de l'hydrogène,

ou encore
3/

- A représente un cycle (sous forme d'un radical divalent) aryle ou aralkyle ayant de 6 à 12 atomes de carbone, notamment de 6 à 8 atomes de carbone, éventuellement substitué par les substituants ci-dessus cités et/ou éventuellement comprenant 1 ou plusieurs hétéroatomes et/ou éventuellement substituée par 1 ou plusieurs halogènes et/ou radicaux hydroxy;
- R et R', identiques ou différents, représentent un atome d'hydrogène ou une chaîne hydrocarbonée saturée ou insaturée, linéaire, ramifiée ou cyclique, de préférence saturée linéaire, comprenant de 6 à 18 atomes de carbone, notamment de 10 à 16 atomes de carbone, de préférence comprenant de 11 à 13 atomes de carbone, plus préférentiellement ayant 11 atomes de carbone, à la condition que R et R' soient différents de l'hydrogène.

Notamment, le radical A peut représenter un radical divalent de type cyclohexylène (en particulier 1,2-cyclohexylène, 1,3-cyclohexylène, 1,4-cyclohexylène ; de préférence 1,2-cyclohexylène), éthylène, propylène, isopropylène, butylène, iso-butylène, pentylène, hexylène, dodécylène, dodécanylène, benzylène, phénylène, méthyl-phénylène, bis-phénylène ou napththalène ; de préférence A peut être un radical divalent de type cyclohexylène, éthylène, propylène, isopropylène, dodécylène, méthyl-phénylène .

Les radicaux R et R' peuvent, indépendamment l'un de l'autre, être choisis parmi les radicaux pentyle, hexyle, décyle, undécyle, dodécyle, pentadécyle, hexadécyle, heptadécyle, octadécyle, 3-dodécyloxyproprionyle, 3-octadécyloxyproprionyle, 3-dodécyloxypentyle, 3-octadécyloxypentyle, 11-hydroxyheptadécyle. De préférence, R et R' peuvent, indépendamment l'un de l'autre, être choisis parmi les radicaux décyle, undécyle, dodécyle.

Avantageusement, R et R' sont identiques.

Lorsque le radical A est cyclique, en particulier est un cyclohexylène, les radicaux R-CO-NH- et R'-CO-NH- peuvent être en position ortho, méta ou para ; par ailleurs, ces radicaux peuvent être en position cis ou trans l'un par rapport à l'autre. Le composé de formule (I) peut d'ailleurs comprendre un mélange du composé cis et des composés trans (mélange racémique ou des énantiomères 1R, 2R ou 1S, 2S, ou leurs mélanges en proportion variable). La stéréochimie du composé de formule (I) correspond en fait à la stéréochimie de la diamine de formule $H_2N$-A-$NH_2$ utilisée lors de la préparation des composés, préparation décrite ci-après.

Préférentiellement, les composés de formule (I) sont choisis parmi les composés répondant à l'une des formules suivantes :

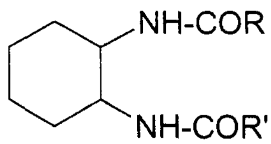

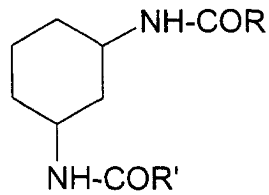

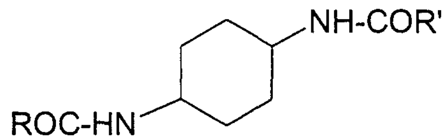

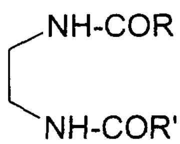

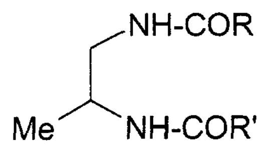

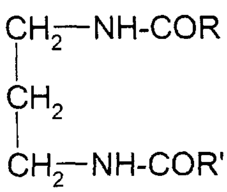

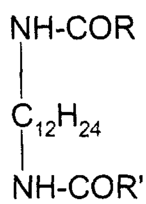

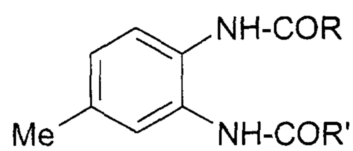

dans laquelle R et R' ont les mêmes significations que ci-dessus.

Parmi les composés susceptibles d'être employés dans le cadre de l'invention, on peut citer :

- le N,N'-bis (dodécanoyl)-1,2-diaminocyclohexane, en particulier sous forme trans (composé de formule (I) avec R=R' = n-$C_{11}H_{23}$ et A = radical divalent 1,2-cyclohexylène, nommé également acide (2-dodécanoylamino-cyclohexyl)-amide dodécanoïque). Ce composé est notamment décrit dans Hanabusa, K ; Angew.Chem., 108, 1997, 17, pages 2086-2088.

- le N,N'-bis (dodécanoyl)-1,3-diaminocyclohexane, en particulier sous forme trans (composé de formule (I) avec R=R'=n-$C_{11}H_{23}$ et A = radical divalent 1,3-cyclohexylène, nommé également acide (3-dodécanoylamino-cyclohexyl)-amide dodécanoïque)

- le N,N'-bis (dodécanoyl)-1,4-diaminocyclohexane, en particulier sous forme trans (composé de formule (I) avec R= n-$C_{11}H_{23}$ et A = radical divalent 1,4-cyclohexylène, nommé également acide (4-dodécanoylamino-cyclohexyl)-amide dodécanoïque)

- le N,N'-bis (dodécanoyl)-1,2-éthylènediamine
(composé de formule (I) avec R=R'=n-$C_{11}H_{23}$ et A = radical divalent 1,2-éthylène, nommé également acide (2-dodécanoylamino-ethyl)-amide dodécanoïque)

- le N,N'-bis (dodécanoyl)-1-méthyl-1,2-éthylènediamine
  (composé de formule (I) avec R=R'=n-$C_{11}H_{23}$ et A = radical divalent 1-méthyl-1,2-éthylène, nommé également acide (2-dodécanoylamino-2-méthyl-éthyl)-amide dodécanoïque)

- le N,N'-bis (dodécanoyl)-1,3-diaminopropane
  (composé de formule (I) avec R=R'=n-$C_{11}H_{23}$ et A = radical divalent 1,3-propylène, nommé également acide (2-dodécanoylamino-propyl)-amide dodécanoïque)

- le N,N'-bis (dodécanoyl)-1,12-diaminododécane
  (composé de formule (I) avec R=R'=n-$C_{11}H_{23}$ et A = radical divalent 1,12-dodécylène, nommé également acide (2-dodécanoylamino-dodécyl)-amide dodécanoïque)

- le N,N'-bis (dodécanoyl)-3,4-diaminotoluène
  (composé de formule (I) avec R-R'=n-$C_{11}H_{23}$ et A = radical divalent 1-méthyl-3,4-phénylène, nommé également acide (2-dodécanoylamino-4-methyl-phenyl)-amide dodécanoïque).

Les composés de formule (I) peuvent être préparés selon des procédés bien connus de l'homme du métier. En particulier, ils peuvent être obtenus par réaction d'une diamine $H_2N$-A-$NH_2$ avec un chlorure d'acide RCOCl et/ou R'COCl avec R, R' ayant la signification ci-dessus mais différent de l'atome d'hydrogène, dans un milieu solvant organique compatible pour la conduite de la réaction (on utilise 1 mole de chlorure d'acide pour 1 mole de diamine si l'on veut obtenir un composé de formule (I) ayant un seul groupement R différent de l'atome d'hydrogène, ou bien 2 moles de chlorure d'acide RCOCl et/ou R'COCl si l'on veut obtenir un composé de formule (II) avec R et R' différents de l'atome d'hydrogène). La réaction est de préférence effectuée en présence d'une base apte à neutraliser la formation de HCl libéré au cours de la réaction. Le diamide formé est extrait du milieu réactionnel selon les techniques d'extraction classiques bien connues de l'homme du métier.
Le composé de formule (I) est de préférence présent dans la composition en une quantité aisément déterminable par l'homme du métier en fonction de l'effet recherché, et qui peut être comprise entre 1 et 40% en poids, par exemple entre 2 et 15% en poids par rapport au poids total de la composition, et encore mieux entre 4 et 12% en poids, voire entre 5 et 10% en poids. On a par ailleurs constaté que même l'utilisation d'une faible quantité de composés de formule (I), par exemple de l'ordre de 2-6% en poids, pouvait conduire à une gélification adéquate de la composition selon l'invention. Ceci est dû à un fort pouvoir épaississant des composés de formule (I), qui leur permet d'être efficace à faible concentration, de l'ordre de 4-8% en poids, alors qu'il serait nécessaire d'utiliser 10-20% en poids de gélifiants usuels pour obtenir un résultat équivalent.
Sans être tenu par la présente explication, on a constaté que la structuration, ou gélification, des huiles grâce aux composés de formule (I) pouvait être due à la formation d'amoncellements sous forme de colonnes des molécules de composés de formule (I), d'où la constitution d'un réseau de fibres ou feuillets, constitué par lesdits composés de formule (I) et par les huiles, ledit réseau ne diffractant pas la lumière, d'où une certaine translucidité, voire transparence.
Les composés de formule (I) peuvent notamment être employés, seul ou en mélange, dans une composition qui comprend un milieu physiologiquement acceptable, notamment dans une composition cosmétique qui comprend donc par ailleurs un milieu cosmétiquement acceptable.
Ce milieu physiologiquement acceptable, ses constituants, leur quantité, la forme galénique de la composition et son mode de préparation, peuvent être choisis par l'homme du métier sur la base de ses connaissances générales en fonction du type de composition recherchée.
Généralement, la composition selon l'invention comprend, dans une phase huileuse, au moins une huile, corps gras liquide à température ambiante (25°C), cosmétiquement ou dermatologiquement acceptable.
Ces huiles peuvent être des huiles hydrocarbonées et/ou siliconées et/ou fluorées.
Elles peuvent être d'origine animale, végétale, minérale ou synthétique.
On peut en particulier citer :

- les huiles hydrocarbonées d'origine animale telles que le perhydrosqualène ;
- les huiles hydrocarbonées végétales telles que les triglycérides liquides d'acides gras de 4 à 10 atomes de carbone comme les triglycérides des acides heptanoïque ou octanoïque; les huiles de tournesol, de maïs, de soja, de courge, de pépins de raisin, d'arachide, d'amande douce, de calophyllum, de palme, de sésame, de noisette, d'abricot, de macadamia, de ricin, d'avocat; les triglycérides des acides caprylique/caprique comme ceux vendus par la société Stearineries Dubois ou ceux vendus sous les dénominations Miglyol 810, 812 et 818 par la société Dynamit Nobel; l'huile de jojoba, de beurre de karité ;
- les hydrocarbures linéaires ou ramifiés, d'origine minérale ou synthétique tels que les huiles de paraffine et leurs dérivés, la vaseline, les polydécènes, le poly-isobutène hydrogéné tel que le parléam ;

- les esters et les éthers de synthèse notamment d'acides gras comme les huiles de formule $R_3COOR_4$ dans laquelle $R_3$ représente le reste d'un acide gras supérieur comportant de 7 à 29 atomes de carbone et $R_4$ représente une chaîne hydrocarbonée contenant de 3 à 30 atomes de carbone comme par exemple l'huile de Purcellin (octanoate de cétostéaryle), le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; le trimellitate de tridécyle ;
- des alcools gras ayant de 12 à 26 atomes de carbone comme l'octyldodécanol, le 2-butyloctanol, le 2-hexyldécanol, le 2-undécylpentadécanol, l'alcool oléique ;
- les huiles fluorées partiellement hydrocarbonées et/ou siliconées;
- les huiles siliconées comme les polyméthylsiloxanes (PDMS) volatiles ou non, linéaires ou cycliques; les alkyidiméthicones; les silicones modifiées par des groupements aliphatiques et/ou aromatiques, éventuellement fluorés, ou par des groupements fonctionnels tels que des groupements hydroxyles, thiols et/ou amines; les huiles siliconées phénylées telles que les polyphénylméthylsiloxanes ou les phényltriméthicones.
- leurs mélanges.

Les huiles employées peuvent être volatiles et/ou non volatiles. Par huile volatile, on entend une huile susceptible de s'évaporer à température ambiante d'un support sur lequel elle a été appliquée, autrement dit une huile ayant une tension de vapeur mesurable à 25°C et 1 atmosphère, par exemple supérieure à 0 Pa, en particulier allant de $10^{-3}$ à 300 mm de Hg (0,13 Pa à 40.000 Pa).

On peut notamment citer les huiles siliconées volatiles, telles que les silicones volatiles cycliques ou linéaires, et les cyclocopolymères. On peut également citer les huiles volatiles hydrocarbonées telles que les isoparaffines, et les huiles fluorées volatiles.

Dans un mode de réalisation particulier, les huiles volatiles peuvent constituer la majeure partie de la phase huileuse. Ainsi, elles peuvent y être présentes à raison d'au moins 50% en poids, notamment au moins 75% en poids, voire 100% en poids, de ladite phase huileuse.

Dans un autre mode préféré de réalisation, la phase huileuse peut comprendre des huiles de silicone, notamment en quantité importante de l'ordre de 40-80% en poids de la phase huileuse, en particulier de l'ordre de 60-75% en poids; la phase huileuse peut également comprendre 100% en poids d'huile siliconée, tout en restant parfaitement stable et solide.

Les huiles peuvent être présentes dans la composition à raison de 5 à 99% en poids du poids total de la composition, de préférence de 20 à 75% en poids.

La composition selon l'invention se présente préférentiellement sous forme solide. On entend par là qu'on n'observe aucun affaissement de la composition en dehors du récipient la comprenant, en l'absence de stimulation mécanique ou thermique (chauffage notamment).

La composition présente un comportement viscoélastique classique d'une composition de type solide.

Par ailleurs, la dureté de la composition selon l'invention est de préférence telle que la composition est autoportée et peut se déliter aisément pour former un dépôt satisfaisant sur la peau et les lèvres. Cette dureté peut être comprise entre 0,04 N et 3 N, de préférence entre 0,1 et 2,5 N, notamment entre 0,2 et 2N. Cette dureté peut être mesurée selon une méthode de pénétration d'une sonde dans ladite composition et en particulier à l'aide d'un analyseur de texture (par exemple-TA-XT2 de chez Rhéo) équipé d'un cône en acrylique d'angle au sommet de 45°. La mesure de dureté est effectuée à 22°C au centre de 5 échantillons de ladite composition selon la méthode décrite dans les exemples.

De manière avantageuse, cette composition comprend peu, voire pas, de cire, tout en conservant une solidité/rigidité/dureté adéquate. On entend par là que la composition comprend moins de environ 5% en poids de cire, par rapport au poids total de la composition, de préférence moins de 2% en poids, voire moins de 0,5% en poids de cire. Préférentiellement, la composition ne contient pas de cires (soit 0%).

Une cire, au sens de la présente invention, est un composé gras lipophile, solide à température ambiante (environ 25°C), à changement d'état solide/liquide réversible, ayant une température de fusion supérieure à environ 40°C pouvant aller jusqu'à 200°C, et présentant à l'état solide une organisation cristalline anisotrope. Les cires, au sens de la demande, sont celles généralement utilisées dans les domaines cosmétique et pharmaceutique.

Elles sont notamment naturelles d'origine animale, végétale ou minérale, comme la cire d'abeilles, la cire de Montan, la cire de Carnauba, la cire de Candellila, la cire de Chine, la cire de lin, la cire de sapin, la cire de coton, la cire d'Ouricoury, la cire de lignite, la cire de son de riz, la cire de canne à sucre, la cire du Japon, la cire de fibres de liège.

On peut également citer les cires de paraffine, les cires microcristallines, la cire de lanoline, les ozokérites, les huiles hydrogénées ayant une température de fusion supérieure à environ 40°C comme l'huile de jojoba hydrogénée, les cires de polyéthylène issues de la polymérisation de l'éthylène, les cires obtenues par synthèse de Fischer-Tropsch, les esters d'acide gras et les glycérides ayant une température de fusion supérieure à environ 40°C, les cires de silicone comme les alkyle, alcoxy et/ou esters de poly(di)méthylsiloxane solide à 40°C.

La composition selon l'invention peut comprendre par ailleurs les constituants usuellement utilisés dans le type d'application envisagé. Elle peut comprendre un ou plusieurs solvants organiques, notamment choisis parmi:

- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol ;
- les éthers de propylène glycol liquides à température ambiante tels que le monométhyléther de propylène glycol, l'acétate de monométhyléther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde.

Il est en outre possible d'incorporer dans la composition selon l'invention une phase hydrophile, notamment en une quantité de 0-10% en poids par rapport au poids total de la composition, et mieux de 1-5% en poids, pouvant comprendre des actifs hydrophiles et/ou des gélifiants hydrophiles. Elle peut notamment comprendre des hydratants tels que la glycérine.

Avantageusement, la composition comprend une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 40% du poids total de la composition, de préférence de 5 à 25% en poids.

Ainsi, la composition peut comprendre une phase particulaire, généralement présente à raison de 0-30% en poids, de préférence 0-20% en poids, et qui peut comprendre des pigments et/ou des nacres et/ou des charges habituellement utilisés dans les compositions cosmétiques. Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.

Les pigments peuvent être blancs ou colorés, minéraux et/ou organiques, de taille micrométrique ou nanométrique. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.

Parmi les nacres envisageables, on peut citer le mica recouvert d'oxyde de titane, d'oxyde de fer, de pigment naturel ou d'oxychlorure de bismuth ainsi que le mica titane coloré.

Les charges peuvent être minérales ou de synthèse, lamellaires ou non lamellaires. On peut citer le talc, le mica, la silice, le kaolin, les poudres de Nylon et de polyéthylène, le Téflon, l'amidon, le micatitane, la nacre naturelle, le nitrure de bore, les microsphères telles que l'Expancel (Nobel Industrie), le polytrap (Dow Corning) et les microbilles de résine de silicone (Tospearls de Toshiba, par exemple).

La composition selon l'invention peut comprendre en outre tout additif usuellement utilisé dans le domaine considéré, notamment le domaine cosmétique, tel que des antioxydants, des parfums, des colorants, des huiles essentielles, des conservateurs, des actifs cosmétiques, des vitamines, des acides gras essentiels, des sphingolipides, des composés auto-bronzants tels que la DHA, des filtres solaires, des tensioactifs, des polymères. Ces additifs peuvent être présents dans la composition à raison de 0-10% en poids.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention sont destinées à être appliquées sur la peau du visage et du corps, sur les muqueuses et/ou sur les fibres kératiniques telles que les ongles, les cils ou les cheveux.

Elles peuvent se présenter sous toutes les formes galéniques envisageables, telles que gel huileux, comprenant éventuellement de l'eau, solide ou souple; émulsion solide ou gélifiée, huile-dans-eau, eau-dans-huile ou multiple; dispersion d'huile dans l'eau; système multiphases notamment biphase. Elles peuvent avoir l'aspect d'une crème,

d'une pommade, d'une pâte souple, d'un onguent, d'un solide coulé ou moulé et notamment d'un stick.

Elles peuvent notamment se présenter sous forme de stick ou de coupelle; et en particulier sous forme d'un gel rigide anhydre transparent, et plus spécialement sous forme de stick anhydre translucide ou transparent.

La gélification de l'huile est telle que l'on peut obtenir une structure rigide sous forme d'un bâton ou d'un stick. Ces bâtons lorsqu'ils sont colorés permettent, après application, d'obtenir un dépôt homogène en couleur.

Ces compositions trouvent notamment une application comme composition d'hygiène corporelle, par exemple sous forme de sticks déodorants; comme composition capillaire, par exemple comme stick de coiffage ou stick de maquillage des cheveux; comme composition de maquillage de la peau du visage ou du corps, ou des muqueuses, par exemple comme rouge à lèvres, fond de teint coulé en stick ou en coupelle, fard à joues ou paupières, base fixante à appliquer sur un rouge à lèvres classique, stick anti-cernes, brillant à lèvres, eye-liner, mascara, produits de tatouage éphémère; comme composition de soin de la peau ou des muqueuses, par exemple comme baume ou base de soin pour les lèvres, onguent pour le corps, crème de soin journalier; comme composition solaire ou auto-bronzante.

Ces compositions trouvent une application toute particulière comme composition de maquillage ou de soin non transfert, notamment comme rouge à lèvre ou fond de teint.

L'invention est illustrée plus en détail dans les exemples suivants.

Méthode de mesure de la dureté des sticks

La dureté est mesurée à l'aide d'un analyseur de texture TA-XT2 (société Rhéo), à 22°C, en utilisant un cône lisse en acrylique, d'angle au sommet 45° et de hauteur totale supérieure à la distance de pénétration. Le cône pénètre à l'intérieur de l'échantillon d'une distance de 5 mm, à une vitesse de 2 mm/s. Il est ensuite maintenu immobile pendant 300 s, puis retiré de l'échantillon à une vitesse de 2 mm/s. La force exercée par l'échantillon sur le corps de mesure est enregistrée en continu. La force maximale est détectée à la fin de la phase de pénétration. Cette valeur de force reflète la dureté de l'échantillon.

Méthode de mesure de la transparence ou la translucidité des sticks

La mesure de la transparence ou de la translucidité est effectuée par mesure de la transmittance, soit le pourcentage de lumière transmise à travers un échantillon donné, dans le domaine des longueurs d'onde correspondant au domaine visible, soit entre 400 et 900 nm.

Cette transmittance est mesurée en continu au travers d'un échantillon d'huile épaissie, placé dans une cuve de verre de chemin optique 1 cm, par différence avec un échantillon dit de référence contenant la même huile pure.

L'instrument de mesure est un spectrophotomètre PERKIN-ELMER Lambda UV-Vis.

La composition étudiée (composé dans l'huile) est chauffée jusqu'à ce qu'elle soit sous forme d'un fluide homogène et est versée directement dans la cuve de mesure. La cuve est maintenue à température ambiante jusqu'au refroidissement de son contenu. On place ensuite la cuve dans l'appareil, la cuve de référence contenant de l'huile pure étant également placée dans l'appareil.

On mesure la transmittance entre 400 et 900 nm.

Préparation-type des composés

Les composés de formule (I) pour lesquels R = R' peuvent être préparé selon les 2 procédés suivants :

1) Premier procédé :

On dissout la diamine et deux équivalents de triéthylamine dans 50 ml de tétrahydrofuranne. On ajoute deux équivalents de chlorure d'acyle en solution dans le THF, on chauffe le mélange réactionnel au reflux du tétrahydrofuranne en suivant la disparition du chlorure d'acyle par spectroscopie infra-rouge (le plus classiquement, deux heures).

On filtre la solution du précipitat, on concentre la phase organique et on réalise une extraction liquide/liquide du composé solide obtenu. La phase organique est ensuite séchée puis concentrée et le produit solide obtenu est recristallisé.

2) Deuxième procédé :

A une solution de 2 moles de chlorure d'acide RCOCl dans un solvant organique S tel que le toluène, le chloroforme, le tétrahydrofurane, on additionne 1 mole de diamine $H_2N\text{-}A\text{-}NH_2$ et 2 moles d'amine tertiaire telle que la triéthylamine préalablement dissoute dans le solvant organique S. Après addition, le milieu réactionnel est chauffé à reflux le temps nécessaire à la disparition du chlorure d'acide et de la diamine (temps de réaction d'environ 2 heures

à 24 heures). L'addition des réactifs peut être également inversée, c'est-à-dire que l'on additionne le chlorure d'acide à une solution de diamine comprenant l'amine tertiaire dans les même proportions relatives indiquées précédemment. Le diamide formé est extrait du milieu réactionnel selon les techniques d'extraction bien connues de l'homme du métier.

Exemple 1

On prépare selon le procédé ci-dessus décrit un composé correspondant à la formule (I) dans laquelle les deux radicaux R et R' représentent une chaîne saturée linéaire à 11 atomes de carbone, le radical A est un cycle saturé à 6 atomes de carbone, et les radicaux RCONH et RCONH' sont en position ortho trans ; ce composé est le trans-N, N'-bis (dodécanoyl)-1,2-diaminocyclohexane.
On mélange sous agitation, à température ambiante :

- 200 mg de composé trans-N,N'-bis (dodécanoyl)-1,2-diaminocyclohexane et
- 10 ml de paraffine fluide vendue sous la dénomination Huile de Parléam par la société NIPPON OIL FATS, soit un mélange à 2% en composé.
  Le mélange est chauffé à 120°C sous agitation, jusqu'à homogénéisation. Il devient alors transparent, homogène et fluide. On laisse alors le mélange homogène refroidir lentement jusqu'à température ambiante.
  On obtient alors une composition translucide, solide et dure, qui ne s'affaisse pas en dehors du récipient, en l'absence de toute stimulation mécanique ou thermique. Cette composition peut être étalée par simple pression et permet l'obtention d'un film huileux et homogène.

On mesure la dureté du stick et on obtient le résultat suivant : 0,329 N.
On mesure la transparence du stick : la transmittance varie de manière continue quasi-linéaire, de 3,7% à 400 nm à 38% à 900 nm (valeur maximale).
Ceci correspond bien à une composition translucide

Exemple 2

On mélange sous agitation, à température ambiante :

- 200 mg de composé préparé à l'exemple 1, et
- 10 ml d'ester gras triméllitate de tridécyle, soit un mélange à 2% en composé.
  Le mélange est chauffé à 120°C sous agitation, jusqu'à homogénéisation. Il devient alors transparent, homogène et fluide. On laisse alors le mélange homogène refroidir lentement jusqu'à température ambiante.
  On obtient alors une composition translucide, solide et dure, qui ne s'affaisse pas en dehors du récipient, en l'absence de toute stimulation mécanique ou thermique. Cette composition peut être étalée par simple pression et permet l'obtention d'un film huileux et homogène.

On mesure la dureté du stick et on obtient le résultat suivant : 0,320 N.
On mesure la transparence du stick : la transmittance varie de manière continue quasi-linéaire, de 12% à 400 nm à 45% à 900 nm (valeur maximale).
Ceci correspond bien à une composition translucide claire, voire transparente.

Exemple 3

On mélange sous agitation, à température ambiante :

- 200 mg de composé préparé à l'exemple 1, et
- 10 ml d'huile silicone fluide phényl triméthicone, vendue sous la dénomination DOW CORNING 556 FLUID COSMETIQUE par la société DOW CORNING soit un mélange à 2% en composé.

Le mélange est chauffé à 120°C sous agitation, jusqu'à homogénéisation. Il devient alors transparent, homogène et fluide. On laisse alors le mélange homogène refroidir lentement jusqu'à température ambiante.
On obtient alors une composition transparente, solide et dure, qui ne s'affaisse pas en dehors du récipient, en l'absence de toute stimulation mécanique ou thermique. Cette composition peut être étalée par simple pression et permet l'obtention d'un film huileux et homogène.

On mesure la dureté du stick et on obtient le résultat suivant : 0,470 N.

On mesure la transparence du stick : la transmittance varie de manière continue quasi-linéaire, de 34% à 400 nm à 78% à 900 nm (valeur maximale).

Ceci correspond bien à une composition transparente.

Exemple 4

On mélange 250 mg du composé de l'exemple 1 avec 5 ml de paraffine fluide (huile de Parléam) et 25 mg de pigment (oxydes de fer), sous agitation à température ambiante. Le mélange est chauffé à 120°C jusqu'à homogénéisation. Il devient transparent, coloré, homogène et fluide. On laisse alors le mélange refroidir lentement jusqu'à température ambiante.

On obtient alors une composition solide et colorée, sous la forme d'un stick. Cette composition ne montre pas de séparation du pigment dans le temps. Elle permet l'obtention d'un film huileux et homogène. Cette composition peut être utilisée comme fond de teint ou comme rouge à lèvres.

## Revendications

1. Composition notamment cosmétique ou pharmaceutique se présentant sous forme solide, comprenant une phase huileuse et au moins un composé de formule (I) suivante :

R-CO-NH-A-NH-CO-R'

dans laquelle :

- R et R', identiques ou différents, représentent un atome d'hydrogène ou une chaîne hydrocarbonée saturée ou insaturée, linéaire, ramifiée ou cyclique, comprenant 1 à 22 atomes de carbone, éventuellement substituée par un ou plusieurs groupements choisis parmi les groupements aryle (-$C_6H_5$), ester (-COOR" avec R" étant un groupe alkyl ayant 2 à 12 atomes de carbone), amide (-CONHR" avec R" étant un groupe alkyl ayant 2 à 12 atomes de carbone), uréthanne (-OCONHR" avec R" étant un groupe alkyl ayant 2 à 12 atomes de carbone), urée (- NHCONHR" avec R" étant un groupe alkyl ayant 2 à 12 atomes de carbone); et/ou éventuellement comprenant 1 à 3 hétéroatomes choisi parmi O, S et N; et/ou éventuellement substituée par 1 à 4 atomes d'halogènes, notamment de fluor et/ou par 1 à 3 radicaux hydroxy,
à la condition que R et R' soient différents de l'hydrogène,
et
- A représente une chaîne hydrocarbonée, saturée ou insaturée, linéaire, cyclique ou ramifiée, ayant 1 à 18 atomes de carbone, notamment 2 à 12 atomes de carbone, éventuellement substitué par un ou plusieurs groupements choisis parmi les groupements aryle (-$C_6H_5$), ester (-COOR" avec R" étant un groupe alkyl ayant 2 à 12 atomes de carbone), amide (-CONHR" avec R" étant un groupe alkyl ayant 2 à 12 atomes de carbone), uréthanne (-OCONHR" avec R" étant un groupe alkyl ayant 2 à 12 atomes de carbone), urée (-NHCONHR" avec R" étant un groupe alkyl ayant 2 à 12 atomes de carbone); et/ou éventuellement comprenant 1 à 3 hétéroatomes choisi parmi O, S et N ; et/ou éventuellement substituée par 1 à 4 atomes d'halogènes, notamment de fluor et/ou par 1 à 3 radicaux hydroxy.

2. Composition selon la revendication 1 , **caractérisée par le fait que** A représente une chaîne hydrocarbonée, saturée ou insaturée, linéaire, cyclique ou ramifiée, ayant de 4 à 10 atomes de carbone.

3. Composition selon la revendication 1 ou 2, **caractérisée par le fait que** A représente une chaîne hydrocarbonée saturée cyclique ayant de 4 à 10 atomes de carbone.

4. Composition selon la revendication 1 ou 2, **caractérisée par le fait que** A désigne un radical divalent choisi parmi les radicaux cyclohexylène, éthylène, propylène, dodécylène, méthyl-phénylène.

5. Composition selon l'une quelconque des revendications précédentes, **caractérisée par le fait que** R et R', identiques ou différents, représentent une chaîne hydrocarbonée saturée ou insaturée, linéaire, ramifiée ou cyclique, comprenant de 6 à 18 atomes de carbone.

6. Composition selon l'une quelconque des revendications précédentes, **caractérisée par le fait que** R et R', identiques ou différents, représentent une chaîne hydrocarbonée saturée ou insaturée, linéaire, ramifiée ou cyclique, comprenant de 10 à 14 atomes de carbone.

7. Composition selon l'une quelconque des revendications précédentes, **caractérisée par le fait que** R et R', identiques ou différents, représentent une chaîne hydrocarbonée linéaire saturée comprenant de 10 à 14 atomes de carbone.

8. Composition selon l'une quelconque des revendications 1 à 6, **caractérisée par le fait que** R et R', identiques ou différents, représentent une chaîne hydrocarbonée saturée ou insaturée, linéaire, ramifiée ou cyclique, comprenant de 11 à 13 atomes de carbone.

9. Composition selon l'une quelconque des revendications précédentes, **caractérisée par le fait que** R et R', identiques ou différents, représentent une chaîne hydrocarbonée linéaire saturée comprenant de 11 à 13 atomes de carbone.

10. Composition selon l'une quelconque des revendications précédentes, **caractérisée par le fait que** R et/ou R' représentent une chaîne hydrocarbonée linéaire saturée comprenant 11 atomes de carbone.

11. Composition selon l'une des revendication précédentes, **caractérisée par le fait que** R et R' sont identiques.

12. Composition selon la revendication 1, dans laquelle les composés répondent à la formule (I) dans laquelle :

- A représente un radical divalent d'un cycle hydrocarboné, saturé ou insaturé mais non aromatique, éventuellement ramifié, ayant de 4 à 12 atomes de carbone, éventuellement substituée par un ou plusieurs groupements choisis parmi les groupements aryle ($-C_6H_5$), ester (-COOR" avec R" étant un étant un groupe alkyl ayant 2 à 12 atomes de carbone), amide (-CONHR" avec R" étant un groupe alkyl ayant 2 à 12 atomes de carbone), uréthanne (-OCONHR" avec R" étant un groupe alkyl ayant 2 à 12 atomes de carbone), urée (-NHCONHR" avec R" étant un groupe alkyl ayant 2 à 12 atomes de carbone)
  et/ou éventuellement comprenant 1 ou plusieurs hétéroatomes et/ou éventuellement substituée par 1 ou plusieurs halogènes et/ou radicaux hydroxy;
- R et R', identiques ou différents, représentent un atome d'hydrogène ou une chaîne hydrocarbonée saturée ou insaturée, linéaire, ramifiée ou cyclique, de préférence saturée linéaire, comprenant de 10 à 16 atomes de carbone, à la condition que R et R' soient différents de l'hydrogène.

13. Composition selon la revendication 12, **caractérisée par le fait que** A représente un radical divalent d'un cycle hydrocarboné, saturé ou insaturé mais non aromatique, ayant de 5 à 7 atomes de carbone.

14. Composition selon la revendication 12, **caractérisée par le fait que** A désigne un radical divalent d'un cycle hydrocarboné saturé ayant de 4 à 12 atomes de carbone.

15. Composition selon l'une des revendications 12 à 14, **caractérisée par le fait que** A désigne un radical divalent d'un cycle hydrocarboné saturé ayant de 5 à 7 atomes de carbone.

16. Composition selon l'une des revendications 12 à 15, **caractérisée par le fait que** A désigne un radical cyclohexylène.

17. Composition selon l'une des revendications 12 à 16, **caractérisée par le fait que** A désigne un radical 1,2-cyclohexylène.

18. Composition selon l'une quelconque des revendications 12 à 17, **caractérisée par le fait que** R et R', identiques ou différents, désignent une chaîne hydrocarbonée saturée ou insaturée, linéaire, ramifiée ou cyclique, de préférence saturée linéaire, comprenant de 10 à 14 atomes de carbone.

19. Composition selon l'une quelconque des revendications 12 à 17, **caractérisée par le fait que** R et R', identiques ou différents, représentent une chaîne hydrocarbonée saturée linéaire comprenant de 10 à 16 atomes de carbone.

20. Composition selon l'une quelconque des revendications 12 à 18, **caractérisée par le fait que** R et R', identiques

ou différents, désignent une chaîne hydrocarbonée saturée linéaire comprenant de 10 à 14 atomes de carbone.

**21.** Composition selon l'une des revendications 12 à 20, **caractérisée par le fait que** R et/ou R' désignent un chaîne hydrocarbonée linéaire saturée comprenant 11 atomes de carbone.

**22.** Composition selon l'une des revendications 12 à 20, **caractérisée par le fait que** R et R' sont identiques.

**23.** Composition selon la revendication 1, **caractérisée par le fait que** les composés répondent à la formule (I) dans laquelle :

- A représente une chaîne hydrocarbonée saturée, linéaire ou ramifiée, ayant de 2 à 18 atomes de carbone, éventuellement substituée par un ou plusieurs groupements choisis parmi les groupements aryle ($-C_6H_5$), ester (-COOR" avec R" étant un étant un groupe alkyl ayant 2 à 12 atomes de carbone), amide (-CONHR" avec R" étant un groupe alkyl ayant 2 à 12 atomes de carbone), uréthanne (-OCONHR" avec R" étant un groupe alkyl ayant 2 à 12 atomes de carbone), urée (- NHCONHR" avec R" étant un groupe alkyl ayant 2 à 12 atomes de carbone), et/ou éventuellement comprenant 1 ou plusieurs hétéroatomes et/ou éventuellement substituée par 1 ou plusieurs halogènes et/ou radicaux hydroxy;
- R et R', identiques ou différents, représentent un atome d'hydrogène ou une chaîne hydrocarbonée saturée ou insaturée, linéaire, ramifiée ou cyclique, comprenant de 10 à 20 atomes de carbone, à la condition que R et R' soient différents de l'hydrogène.

**24.** Composition selon la revendication 23, **caractérisée par le fait que** A représente une chaîne hydrocarbonée saturée, linéaire ou ramifiée, ayant de 3 à 12 atomes de carbone.

**25.** Composition selon l'une des revendications 23 ou 24, **caractérisée par le fait que** R et R', identiques ou différents, représentent une chaîne hydrocarbonée saturée ou insaturée, linéaire, ramifiée ou cyclique, comprenant de 10 à 20 atomes de carbone.

**26.** Composition selon l'une des revendications 23 à 25, **caractérisée par le fait que** R et R', identiques ou différents, représentent une chaîne hydrocarbonée saturée ou insaturée, linéaire, ramifiée ou cyclique, comprenant de 11 à 18 atomes de carbone.

**27.** Composition selon l'une des revendications 23 à 26, **caractérisée par le fait que** R et R', identiques ou différents, représentent une chaîne hydrocarbonée saturée ou insaturée, linéaire, ramifiée ou cyclique, comprenant de 11 à 13 atomes de carbone.

**28.** Composition selon l'une des revendications 23 à 26, **caractérisée par le fait que** R et R', identiques ou différents, représentent une chaîne hydrocarbonée saturée linéaire comprenant de 10 à 20 atomes de carbone.

**29.** Composition selon l'une des revendications 23 à 26, 28, **caractérisée par le fait que** R et R', identiques ou différents, représentent une chaîne hydrocarbonée saturée linéaire comprenant de 11 à 18 atomes de carbone.

**30.** Composition selon l'une des revendications 23 à 29, **caractérisée par le fait que** R et R', identiques ou différents, représentent une chaîne hydrocarbonée saturée linéaire comprenant de 11 à 13 atomes de carbone.

**31.** Composition selon l'une des revendications 23 à 30, **caractérisée par le fait que** R et/ou R' représentent une chaîne hydrocarbonée saturée linéaire comprenant 11 atomes de carbone.

**32.** Composition selon l'une des revendications 23 à 31, **caractérisée par le fait que** R et R' sont identiques.

**33.** Composition selon la revendication 1, dans laquelle les composés répondent à la formule (I) dans laquelle :

- A représente un cycle aryle ou aralkyle ayant de 6 à 12 atomes de carbone, éventuellement substitué par un ou plusieurs groupements choisis parmi les groupements ester (-COOR" avec R" étant un étant un groupe alkyl ayant 2 à 12 atomes de carbone), amide (-CONHR" avec R" étant un groupe alkyl ayant 2 à 12 atomes de carbone), uréthanne (-OCONHR" avec R" étant un groupe alkyl ayant 2 à 12 atomes de carbone), urée (-NHCONHR" avec R" étant un groupe alkyl ayant 2 à 12 atomes de carbone)
  et/ou éventuellement comprenant 1 ou plusieurs hétéroatomes et/ou éventuellement substituée par 1 ou plusieurs halogènes et/ou radicaux hydroxy;

- R et R', identiques ou différents, représentent un atome d'hydrogène ou une chaîne hydrocarbonée saturée ou insaturée, linéaire, ramifiée ou cyclique, comprenant de 6 à 18 atomes de carbone, à la condition que R et R' soient différents de l'hydrogène.

**34.** Composition selon la revendication 33, **caractérisée par le fait que** A représente un cycle aryle ou aralkyle ayant de 6 à 8 atomes de carbone.

**35.** Composition selon la revendication 33 ou 34, **caractérisée par le fait que** R et R', identiques ou différents, représentent une chaîne hydrocarbonée saturée ou insaturée, linéaire, ramifiée ou cyclique, comprenant de 10 à 16 atomes de carbone.

**36.** Composition selon l'une des revendications 33 à 35, **caractérisée par le fait que** R et R', identiques ou différents, représentent une chaîne hydrocarbonée saturée ou insaturée, linéaire, ramifiée ou cyclique, comprenant de 11 à 13 atomes de carbone.

**37.** Composition selon l'une des revendications 33 à 34, **caractérisée par le fait que** R et R', identiques ou différents, représentent une chaîne hydrocarbonée saturée linéaire comprenant de 6 à 18 atomes de carbone.

**38.** Composition selon l'une des revendications 33 à 35, 37, **caractérisée par le fait que** R et R', identiques ou différents, représentent une chaîne hydrocarbonée saturée linéaire comprenant de 10 à 16 atomes de carbone.

**39.** Composition selon l'une des revendications 33 à 38, **caractérisée par le fait que** R et R', identiques ou différents, représentent une chaîne hydrocarbonée saturée linéaire comprenant de 11 à 13 atomes de carbone.

**40.** Composition selon l'une des revendications 33 à 39, **caractérisée par le fait que** R et R', identiques ou différents, représentent une chaîne hydrocarbonée saturée linéaire comprenant 11 atomes de carbone.

**41.** Composition selon l'une des revendications 33 à 40, **caractérisée par le fait que** R et R' sont identiques.

**42.** Composition selon l'une des revendications 1, 6 à 11, 23, 25 à 32, dans laquelle le radical A représente un radical divalent de type éthylène, propylène, isopropylène, butylène, isobutylène, pentylène, hexylène, dodécylène, dodécanylène.

**43.** Composition selon l'une des revendications 1, 6 à 11, 33, 35 à 41, dans laquelle A représente un radical divalent choisi parmi les radicaux benzylène, phénylène, méthyl-phénylène, bis-phénylène ou napththalène.

**44.** Composition selon l'une des revendications 1 à 3, 12 à 17, 23, 24, dans laquelle les radicaux R et R' sont, indépendamment l'un de l'autre, choisis parmi les radicaux pentyle, hexyle, décyle, undécyle, dodécyle, pentadécyle, hexadécyle, heptadécyle, octadécyle, 3-dodécyloxyproprionyle, 3-octadécyloxyproprio-nyle, 3-dodécyloxypentyle, 3-octadécyloxypentyle, 11-hydroxyheptadécyle.

**45.** Composition selon l'une des revendications 1, 2, dans laquelle les composés de formule (I) sont choisis parmi les composés répondant à l'une des formules suivantes :

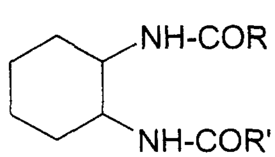

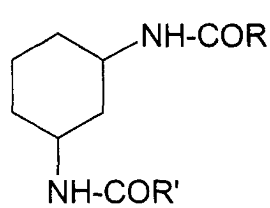

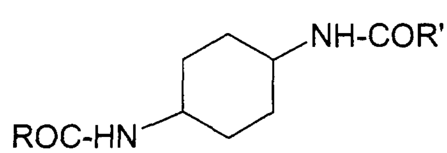

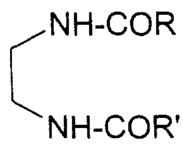

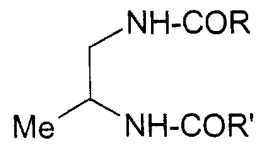

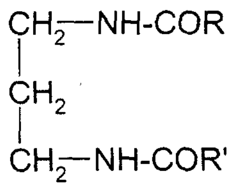

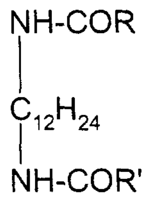

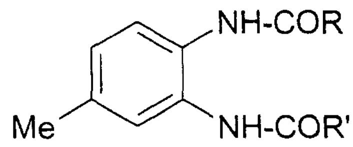

dans laquelle R et R' ont la signification donnée dans la revendication 1.

**46.** Composition selon la revendication précédente, **caractérisée par le fait que** R et R', identiques ou différents, représentent une chaîne hydrocarbonée saturée ou insaturée, linéaire, ramifiée ou cyclique, comprenant de 6 à 18 atomes de carbone.

**47.** Composition selon la revendication 45 ou 46, **caractérisée par le fait que** R et R', identiques ou différents, représentent une chaîne hydrocarbonée saturée ou insaturée, linéaire, ramifiée ou cyclique, comprenant de 10 à 14 atomes de carbone.

**48.** Composition selon l'une des revendications 46 ou 47, **caractérisée par le fait que** R et R', identiques ou différents, représentent une chaîne hydrocarbonée linéaire saturée comprenant de 10 à 14 atomes de carbone.

**49.** Composition selon l'une quelconque des revendications 46 ou 47, **caractérisée par le fait que** R et R', identiques ou différents, représentent une chaîne hydrocarbonée saturée ou insaturée, linéaire, ramifiée ou cyclique, comprenant de 11 à 13 atomes de carbone.

**50.** Composition selon l'une quelconque des revendications 46 à 49, **caractérisée par le fait que** R et R', identiques ou différents, représentent une chaîne hydrocarbonée linéaire saturée comprenant de 11 à 13 atomes de carbone.

**51.** Composition selon l'une quelconque des revendications 46 à 50, **caractérisée par le fait que** R et/ou R' représentent une chaîne hydrocarbonée linéaire saturée comprenant 11 atomes de carbone.

**52.** Composition selon l'une des revendication 46 à 50, **caractérisée par le fait que** R et R' sont identiques.

**53.** Composition selon l'une des revendications 1 à 22, 46 à 52, dans laquelle le composé de formule (I) est choisi parmi :

- le N,N'-bis (dodécanoyl)-1,2-diaminocyclohexane
- le N,N'-bis (dodécanoyl)-1,3-diaminocyclohexane
- le N,N'-bis (dodécanoyl)-1,4-diaminocyclohexane

**54.** Composition selon l'une des revendications 1 à 11, 23 à 32, 46 à 52, dans laquelle le composé de formule (I) est choisi parmi :

- le N,N'-bis (dodécanoyl)-1,2-éthylènediamine
- le N,N'-bis (dodécanoyl)-1-méthyl-1,2-éthylènediamine
- le N,N'-bis (dodécanoyl)-1,3-diaminopropane

- le N,N'-bis (dodécanoyl)-1,12-diaminododécane

**55.** Composition selon l'une des revendications 1 à 11, 33 à 41, 46 à 52, dans laquelle le composé de formule (I) est le N,N'-bis (dodecanoyl)-3,4-diaminoto-luène.

**56.** Composition selon l'une des revendications précédentes, dans laquelle les composés de formule (I) sont présents en une quantité comprise entre 1 et 40% en poids, par exemple entre 2 et 15% en poids par rapport au poids total de la composition, et encore mieux entre 4 et 12% en poids, voire entre 5 et 10% en poids.

**57.** Composition selon l'une des revendications précédentes, dans laquelle la phase huileuse comprend une huile choisie parmi les huiles hydrocarbonées et/ou siliconées et/ou fluorées, d'origine animale, végétale, minérale ou synthétique.

**58.** Composition selon l'une des revendications précédentes, dans laquelle la phase huileuse comprend une huile choisie parmi les huiles hydrocarbonées d'origine animale ; les huiles hydrocarbonées végétales ; les hydrocarbures linéaires ou ramifiés, d'origine minérale ou synthétique ; les esters et les éthers de synthèse ; les esters hydroxylés ; les esters de poly ol ;les alcools gras ayant de 12 à 26 atomes de carbone ; les huiles fluorées partiellement hydrocarbonées et/ou siliconées ; les polyméthylsiloxanes (PDMS) volatiles ou non, linéaires ou cycliques ; les alkyldiméthicones; les silicones modifiées par des groupements aliphatiques et/ou aromatiques, éventuellement fluorés, ou par des groupements fonctionnels tels que des groupements hydroxyles, thiols et/ou amines ; les huiles siliconées phénylées ; leurs mélanges.

**59.** Composition selon l'une des revendications précédentes, dans laquelle la phase huileuse comprend une huile choisie parmi :

- le perhydrosqualène ;
- les triglycérides liquides d'acides gras de 4 à 10 atomes de carbone ; les huiles de tournesol, de maïs, de soja, de courge, de pépins de raisin, d'arachide, d'amande douce, de calophyllum, de palme, de sésame, de noisette, d'abricot, de macadamia, de ricin, d'avocat; les triglycérides des acides caprylique/caprique ; l'huile de jojoba, de beurre de karité ;
- les huiles de paraffine, la vaseline, les polydécènes, l'huile de Purcellin, le poly-isobutène hydrogéné ;
- le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; le dioctanoate de propylène glycol, le diheptanoate de néopentylglycol, le diisononanoate de diéthylèneglycol ; et les esters du pentaérythritol ; le trimellitate de tridécyle ;
- l'octyldodécanol, le 2-butyloctanol, le 2-hexyldécanol, le 2-undécylpentadécanol, l'alcool oléique ;
- les polyphénylméthylsiloxanes ou les phényltriméthicones.
- leurs mélanges.

**60.** Composition selon l'une des revendications précédentes, dans laquelle la phase huileuse comprend au moins 50% en poids, notamment au moins 75% en poids, voire 100% en poids, d'huiles volatiles.

**61.** Composition selon l'une des revendications 1 à 60, dans laquelle la phase huileuse comprend des huiles de silicone, notamment en quantité de l'ordre de 40-80% en poids de la phase huileuse, en particulier de l'ordre de 60-75% en poids; voire 100% en poids d'huile siliconée.

**62.** Composition selon l'une des revendications précédentes, présentant une dureté comprise entre 0,04 N et 3 N, de préférence entre 0,1 et 2,5 N, notamment entre 0,2 et 2N.

**63.** Composition selon l'une des revendications précédentes, comprenant moins de environ 5% en poids de cire, par rapport au poids total de la composition, de préférence moins de 2% en poids, voire moins de 0,5% en poids de cire.

**64.** Composition selon l'une des revendication 1 à 62, comprenant 0% en poids de cires.

**65.** Composition selon l'une des revendications précédentes, présentant une valeur de transmittance maximum de la lumière, quelle que soit la longueur d'onde comprise entre 400 et 800 nm, à travers un échantillon de 1 cm d'épaisseur, comprise entre 2 et 100%, de préférence d'au moins 50%.

**66.** Composition selon l'une quelconque des revendications précédentes, **caractérisée par le fait qu'**elle comprend au moins un additif choisi parmi les antioxydants, les parfums, les colorants, les huiles essentielles, les conservateurs, les actifs cosmétiques, les vitamines, les acides gras essentiels, les sphingolipides, les composés autobronzants, les filtres solaires, les tensioactifs, les polymères, les pigments, les nacres, les charges, les hydratants.

**67.** Composition selon l'une des revendications précédentes, se présentant sous la forme d'un gel huileux, comprenant éventuellement de l'eau, solide ou souple ; d'une émulsion solide ou gélifiée, huile-dans-eau, eau-dans-huile ou multiple ; d'une dispersion d'huile dans l'eau ; d'un système multiphases ; d'une crème, d'une pommade, d'une pâte souple, d'un onguent, d'un solide coulé ou moulé, d'un stick.

**68.** Composition selon l'une quelconque des revendications précédentes, **caractérisée par le fait qu'**elle se présente sous la forme d'une composition solide.

**69.** Composition selon l'une des revendications précédentes, se présentant sous la forme de sticks déodorants, de stick de coiffage, de stick de maquillage des cheveux; d'une composition de maquillage de la peau du visage ou du corps, de rouge à lèvres, de fond de teint coulé en stick ou en coupelle, de fard à joues ou paupières, de base fixante à appliquer sur un rouge à lèvres classique, de stick anti-cernes, de brillant à lèvres, d'eye-liner, de mascara, de produits de tatouage éphémère, d'une composition de soin de la peau ou des muqueuses, de baume ou base de soin pour les lèvres, d'onguent pour le corps, de crème de soin journalier, d'une composition solaire, d'une composition auto-bronzante.

Office européen des brevets

**RAPPORT PARTIEL DE RECHERCHE EUROPEENNE**
qui selon la règle 45 de la Convention sur le brevet européen est consideré, aux fins de la procédure ultérieure, comme le rapport de la recherche européenne

Numéro de la demande
EP 01 40 1905

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 3 857 960 A (MACKLES, LEONARD) 31 décembre 1974 (1974-12-31)<br>* colonne 1, ligne 16-22 *<br>* colonne 1, ligne 66 - colonne 2, ligne 21 *<br>* exemples 1,2 *<br>* revendications 1,4,6,7 *<br>---<br>-/-- | 1,4,5, 11,23, 25,26, 28,29, 42, 44-46, 56-59, 64,66-69 | A61K7/027<br>A61K7/48 |

DOMAINES TECHNIQUES RECHERCHES (Int.Cl.7)
A61K

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| LA HAYE | 27 septembre 2001 | Bazzanini, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C08)

Office européen des brevets

**RECHERCHE INCOMPLETE**
**FEUILLE SUPPLEMENTAIRE C**

Numéro de la demande
EP 01 40 1905

Revendications ayant fait
l'objet de recherches complètes:
53-55

Revendications ayant fait
l'objet de recherches incomplètes:
1-52, 56-69

Raison pour la limitation de la recherche:

Les revendications 1-52 et 56-59 ont trait à une très grande variété de composés (composés de formule R-CO-NH-A-NH-CO-R'). Un fondement au sens de L'Article 84 CBE et un exposé au sens de l'Article 83 CBE ne peut cependant être trouvé que pour un nombre très restreint de ces composés revendiqués. Dans le cas présent, les revendications manquent à un tel point de fondement et l'exposé de l'invention dans la description est si limité q'une recherche significative couvrant tout le spectre revendiqué est impossible.

En outre les revendications 62 et 65 ont trait à une composition solide définie au moyen des paramètres suivants:
P1: dureté
P2: valeur de transmittance maximum de la lumière.

L'utilisation de ces paramètres est considérée , dans le présent contexte, comme menant à un manque de clarté au sens de l'Article 84 CBE. Il est impossible de comparer les paramètres que le déposant a choisi d'utiliser avec ce qui est révélé dans l'état de la technique. Le manque de clarté qui en découle est tel q'une recherche significative complète est impossible.

Par conséquent, la recherche a été limitée aux parties des revendications ayant un fondement et un exposé suffisant, c'est à dire les parties ayant trait aux composés mentionnés dans les revendications 53-55 (bis-dodecanoyl-amides) et aux composés donnés dans les fromules de la revendication 45, étant R et R' limités aux chaines hydrocarbonnées saturées et linéaires, comprenant 1 à 22 atomes de carbone.

Office européen des brevets

RAPPORT PARTIEL DE RECHERCHE EUROPEENNE

Numero de la demande
EP 01 40 1905

| DOCUMENTS CONSIDERES COMME PERTINENTS | | | CLASSEMENT DE LA DEMANDE (Int.Cl.7) |
|---|---|---|---|
| Catégorie | Citation du document avec indication, en cas de besoin, des parties pertinentes | Revendication concernée | |
| X,D | DATABASE CA 'en ligne! CHEMICAL ABSTRACTS SERVICE, COLUMBUS, OHIO, US; HANABUSA, KENJI ET AL: "Stabilizing compositions containing 1,2-bis(acylamino)cyclohexanes for food, cosmetics, pharmaceuticals, etc." retrieved from STN Database accession no. 129:280780 XP002176460 * abrégé * & JP 10 237034 A (POLA CHEMICAL INDUSTRIES, INC., JAPAN) 8 septembre 1998 (1998-09-08) --- | 1-5, 11-17, 19, 22-26, 28,29, 32, 44-46, 52, 56-59, 63,66-69 | |
| | | | DOMAINES TECHNIQUES RECHERCHES (Int.Cl.7) |
| X,D | DATABASE CA 'en ligne! CHEMICAL ABSTRACTS SERVICE, COLUMBUS, OHIO, US; GOTO, HIROYUKI ET AL: "Solidifying and gelling agents for organic liquid" retrieved from STN Database accession no. 123:173532 XP002176461 * abrégé * & JP 07 138555 A (NISSHIN FINE CHEMICAL KK, JAPAN) 30 mai 1995 (1995-05-30) ----- | 1,2,4,5, 11, 23-26, 28,29, 32,42, 44-46, 52, 56-59, 63,66-69 | |

EPO FORM 1503 03.82 (P04C11)

**ANNEXE AU RAPPORT DE RECHERCHE EUROPEENNE RELATIF A LA DEMANDE DE BREVET EUROPEEN NO.** EP 01 40 1905

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-09-2001

| Document brevet cité au rapport de recherche | | Date de publication | Membre(s) de la famille de brevet(s) | | Date de publication |
|---|---|---|---|---|---|
| US 3857960 | A | 31-12-1974 | CA | 1020091 A1 | 01-11-1977 |
| JP 10237034 | A | 08-09-1998 | AUCUN | | |
| JP 7138555 | A | 30-05-1995 | JP | 2956817 B2 | 04-10-1999 |

EPO FORM P0460

Pour tout renseignement concernant cette annexe : voir Journal Officiel de l'Office européen des brevets, No.12/82